# EUROPEAN PATENT SPECIFICATION

(11) **EP 3 388 097 B1**
(45) Date of publication and mention of the grant of the patent: **03.01.2024**
(21) Application number: 18158300.6
(22) Date of filing: 12.11.2008
(51) Int. Cl.: A61M 5/315, A61M 5/31

(54) **ADMINISTRATION DEVICE COMPRISING DOSE DEVIATION INDICATION UNIT**
VERABREICHUNGSVORRICHTUNG MIT EINER EINHEIT ZUR ANZEIGE VON DOSISABWEICHUNGEN
DISPOSITIF D'ADMINISTRATION COMPRENANT UNE UNITÉ D'INDICATION ET MÉTHODE POUR AFFICHER UNE DOSE AFFICHER UNE DOSE

(30) Priority: 13.11.2007 EP 07021986
(43) Date of publication of application: 17.10.2018
(62) Divisional of application: 08850330.5
(73) Proprietor: Roche Diabetes Care GmbH, 68305 Mannheim (DE); F. Hoffmann-La Roche AG, 4070 Basel (CH)
(72) Inventor: REMDE, Axel, 3432 Lützelflüh (CH)
(74) Representative: Rentsch Partner AG

(56) References cited:
- EP-A- 1 498 067
- WO-A-2007/056592
- WO-A-2007/107431
- DE-U1- 29 904 864

## Description

The present invention is related to an administration device for the administration of a liquid drug in adjustable doses and to a method for setting a dose of a liquid drug.

In the therapy of insulin-dependent diabetes mellitus, pen-shaped administration devices for the self-administration of insulin in adjustable doses are widely used and well known in the art. Such devices may be designed for ejecting a single, pre-installed insulin reservoir and to be disposed after fully emptying the reservoir. Such a device is exemplarily disclosed in WO 97/36625. Other devices are designed for multiple use whit the insulin reservoir being exchanged after emptying. Such devices may be of a more complicated design and may be equipped with an electronics module, e.g., for dose displaying purposes, such as the device disclosed in WO 93/16740. Often several of such administration devices are being used by one patient with, e.g., one administration device generally kept at home and another administration device at the workplace. Furthermore, therapy is often performed with more than one type of insulin, the different types of insulin being administered at different times of day and with different doses. Each of the insulin types is administered with a different administration device. Besides the therapy of insulin-dependent diabetes mellitus, similar devices are used for the administration of other liquid drugs, such as pain relievers or growth hormones.

In US 6482185 an administration device is disclosed, the administration device comprising an injection pen adapted for the self-administration of insulin in adjustable doses and an external display apparatus for enlarged displaying the injection dose. The display apparatus may further comprise means for entering carbohydrate data and a dose calculator for calculating an insulin dose to be injected. The external display device is bulky and has to be separated from the pen before insulin administration. This results in a number of drawbacks, partly related to user comfort which is known to directly influence acceptance.

When using an administration device having manually operated dose entry means, such as a typical insulin pen, manually setting the dose involves potential user errors, resulting in unintended over administration or under administration of the drug.

According to the disclosure of US 6482185, this problem may be resolved by integrating a drive into the external display apparatus, the drive being operationally coupled to the dose calculator and the injection pen, such that a dose amount calculated by the dose calculator is automatically set. This approach, however, results in a rather complex, bulky and expensive electromechanical system. A further prior art administration device is disclosed in EP 1 498 067 A.

An administration device according to the present invention, which is defined in the independent claim 1, comprises an injection unit, the injection unit comprising manually operated dose setting means and a dosing mechanism. Operating the manually operated dose setting means sets an administration dose amount AD to be administered. An administration device according to the present invention further comprises an electronic indication unit, the indication unit indicating the relation between the administration dose amount AD and a target dose amount TD.

In preferred embodiments, the liquid drug is insulin and the administration device is used for the therapy of diabetes mellitus.

In preferred embodiments, the manually operated dose setting means are realized as dose setting knob, the dose setting knob being rotatable for setting a dose amount. For determining the administration dose amount AD, an encoder is provided in especially preferred embodiments, the encoder transferring the rotation of the dose setting knob in to an electrical signal, the electrical signal being indicative for the administration dose amount AD.

In preferred embodiments, the drug administration is performed after dose setting by pressing down the preferably present dose setting knob.

The indication unit is operatively coupled to or comprised by further electronics components such as a micro controller, an ASIC and/or further electronic circuitry known in the art. In especially preferred embodiments, the indication unit is part of an electronics module of the injection device.

In preferred embodiments, the indication unit comprises a display. In especially preferred embodiments, the display is a liquid crystal display (LCD) adopted to indicate at least numeric values and application specific symbols, but the display may also be adopted to display alphanumeric values and/or be a graphical display.

In some preferred embodiments, the display indicates at least one of the administration dose amount AD, the target dose amount TD and the dose amount deviation ΔD, the dose amount deviation ΔD indicating the deviation between the administration dose amount AD and the target dose amount TD. The dose amount deviation ΔD may especially be the difference between the administration dose amount AD and the target dose amount TD. In some especially preferred embodiments, the display indicates all of the administration dose amount AD, the target dose amount TD and the dose amount deviation ΔD.

In some preferred embodiment, the relation between the administration dose amount AD and the target dose amount TD is indicated by dedicated symbols on the display. In some embodiments, a first symbol is provided to indicate that the administration dose amount AD does not equal the target dose amount TD while a second symbol is provided to indicate that the administration dose amount AD equals the target dose amount TD. In some embodiments, a first arrow symbol and a second arrow symbol are provided, the first arrow symbol indicating that the administration dose amount AD is smaller than the target dose amount TD and the second arrow symbol indication that administration dose amount AD is larger than the target dose amount TD.

In especially preferred embodiments, the display indicates a graphical representation of the dose amount deviation ΔD. For this purpose, the display indicates a dose scale, the dose scale showing a graduation, an administration dose mark, with the administration dose mark indicating the administration dose amount AD, and a target dose mark, with the target dose mark indicating the target dose amount TD. From the relative positions of the administration dose mark and the target dose mark, the dose amount deviation ΔD is easily obtained. In this type of embodiment, equivalence of the administration dose amount AD and the target dose amount TD is visible by an alignment of the administration dose mark and the target dose mark on the scale.

Alternatively or additionally to a display, the indication unit may comprise other kinds of optical indicators, especially luminous indicators, such as light emitting diodes (LEDs). Providing luminous indicators is especially favorable for the usage under inadequate lighting conditions where a display may be hard to read and/or for visually handicapped diabetics.

For example, a single LED may be provided to indicate if the administration dose amount AD equals the target dose or TD or to indicate if the administration dose amount AD does not equal the target dose amount TD. In further embodiments, a multi-color LED is provided, the color of the LED indicating the relation between the administration dose amount AD and the target dose amount TD. For example, a three-colored LED may be red if the deviation between the administration dose amount AD and the target dose amount TD is larger than, e.g., 0.5 IU, yellow if deviation between administration dose amount AD and target dose amount TD is smaller than 0.5 IU and green if the administration dose amount AD equals the target dose amount TD. Alternatively or additionally, a multi-color LED may be provided to indicate if the administration dose amount AD is smaller than the target dose amount TD, equals the target dose amount TD or is larger than the target dose amount TD. In further embodiments, a number of LEDs is provided, the LEDs forming a scale in preferred embodiments.

In some preferred embodiments, the indication unit further comprises at least one of an acoustical indicator, such as a buzzer, and a tactile indicator, such as a pager vibrator. An acoustical indicator and or a tactile indicator may be activated if the administration dose amount AD equals the target dose amount TD or may be the activated if administration dose amount AD does not equal the target dose amount TD. In some embodiments, the preferably provided acoustical indicator and/or tactile indicator also indicates the dose amount deviation ΔD. For example, an acoustical indicator and/or a tactile indicator may be periodically activated with the activation frequency depending on dose amount deviation ΔD or an acoustic indicator may be activated with a frequency and/or intensity depending on the dose amount deviation ΔD. In further embodiments, the relation between the administration dose amount AD and the target dose amount TD is indicated via speech output.

In preferred embodiments, the administration device further comprises a data interface. The data interface may serve several purposes, such as the transmission of configuration data from an external device to the administration device. In especially preferred embodiments, the administration device further stores the dose amounts of administered doses in a history memory together with a time stamp, the time stamp comprising a date portion and a time-of day portion and being generated by a clock circuit of the administration device. The history may be downloaded to an external device via the preferably present data interface. In especially preferred embodiments, the data interface is a bidirectional interface and may especially be a wireless infrared interface and/or a RF interface such as a BLUETOOTH interface. The external device may be, for example, a Personal Computer (PC), a mobile phone, a Personal Digital Assistant (PDA) or an application specific device.

In especially preferred embodiments, a target dose amount TD may be transmitted to the administration device form an external device via the preferably present data interface of the administration device.

In some preferred embodiments, the target dose amount TD is stored in the administration device. In the framework of diabetes therapy, this may be the case, e.g. for an administration device for the administration of long-acting insulin which is to be administered every day at the same time of day and with the same dose amount in order to cover a diabetics basal insulin demand. Similar situations may also occur in other therapies, such as pain therapy, where preset dose amounts of an pain-relieving drug are administered. In some embodiments, several alternative values for the target dose amount TD are stored in the administration device. This may be the case, e.g., if a long-acting insulin is to be administered in different dose amounts at different times of day. For each administration, the target dose amount TD may be manually selected and/or may be automatically selected by the administration device, in dependence, e.g., of the time of day. A target dose to be stored in the administration device may be factory set and/or be transferred to the administration device. In preferred embodiments, the administration device further comprises a data entry means. The data entry means may especially be adapted for the entry of therapy related data. The data entry means may be realized by one or several pushbuttons, but may alternatively or additionally comprise more sophisticated input means, such as a joystick. In especially preferred embodiments, the data entry means comprises a crossbar switch. In especially preferred embodiments, therapy related data entered via the data entry means may be stored in a history memory together with a time stamp indicating the time of entry. In especially preferred embodiments, the history may be downloaded via the preferably present data interface of the administration device to an external device. Therapy related data entered via the preferably present data entry means may especially be downloaded together with the dose amount history.

In the framework of diabetes, therapy the therapy-related data comprise at least either of a carbohydrate amount or a blood glucose value.

In preferred embodiments, the administration device further comprises a dose calculator for calculating a calculated dose amount Dcalc based on therapy related data, wherein the target dose amount TD is set to the calculated dose amount Dcalc.

In the framework of diabetes therapy, the therapy related data may especially be or comprise the carbohydrate amount of food to be taken in by the patient. The calculated dose amount Dclalc for covering an intake of food may be calculated based on a first set of patient-specific and/or time-of-day dependent factors specifying the dose amount of an insulin dose to cover a given carbohydrate intake. The first set of factors is preferably uploaded to the administration device as configuration data form an external device via a data interface but may also be manually entered via data entry means of the administration device. Similarly, an appropriate calculated dose amount Dcalc for correcting an undesirably raised blood glucose value may be calculated by the preferably present dose calculator based on a second set of patient-dependent and/or time-of-day dependent factors specifying the dose amount of an insulin dose required to correct an undesired raise in blood glucose.

In further embodiments, the therapy related data additionally comprise at least either of a level and/or a duration of sportive activity, data related to mental stress, data related to illnesses or data related to the intake or administration of additional drugs.

In the framework of pain therapy, a calculated dose amount Dcalc may be calculated based, at least in part, on a current pain level.

Therapy related data on which the calculation of the calculated dose amount Dcalc is based may be preferably entered via preferably present data entry means, but may additionally or alternatively also be transmitted to the administration device from an external device via a data interface which is preferably comprised by the administration device.

In some especially preferred embodiments, the administration device comprises a blood glucose meter to provide blood glucose values. In addition to or alternatively to the administration device comprising a blood glucose meter, blood glucose values may be transmitted to the administration device from an external blood glucose meter such as a commercially available strip-based blood glucose meter, e.g. ACCU-CHEK^{®} Compact plus or ACCU-CHEK^{®} Aviva.

In the framework of diabetes therapy, the administration device comprises a carbohydrate amount database in especially preferred embodiments. The carbohydrate amount database stores the relative and/or absolute carbohydrate amounts of a number of meals and a meal may be selected from the carbohydrate amount database via the data entry means. This kind of embodiment allows for an especially simple and user-friendly entry of carbohydrate amounts without the need of explicitly calculating or estimating the carbohydrate amount. For meals having a well defined and fixed carbohydrate amount, such as many fast food meals, the absolute carbohydrate amount is preferably stored in the carbohydrate amount database. For meals of variable size, a relative carbohydrate amount is preferably stored in the data base, while an additional information for specifying the meal size may be entered by the patient via the data entry means. The meal size may be either quantitative or semi-quantitative, such as "large", "medium" or "small". In preferred embodiments, the administration device is designed to be, at least in part, handheld while performing a drug administration.

In some especially preferred embodiments, all components of the administration device are enclosed by an elongated housing, especially a pen-shaped housing, or the components of the administration device are comprised by a number of at least two housings, the at least two housings being adopted to be physically connected, resulting in an overall elongated shape, especially a pen-like shape.

In alternative preferred embodiments, the administration device comprises an injection device and an supplementary device. In this type of embodiment, the injection device comprises the injection unit and the indication unit. The injection device housing further comprises an injection device data interface and an injection device housing. The supplementary device comprises an supplementary device data interface and an supplementary device housing. The supplementary device data interface is adapted to transmit a target dose amount TD to injection device data interface. In especially preferred embodiments, the supplementary device further comprises a data entry means for entering therapy related data, which may, e.g., be realized as touch screen. Because only a limited number of elements is required on the injection device according to this type of embodiment, the injection device may be especially slim, lightweight and cheap. In addition, the indication unit may be rather simple and consist, e.g., of a substantially numeric LCD display while the supplementary device may provide a more sophisticated user interface.

In further alternatively preferred embodiments, the administration device also comprises an injection device and a supplementary device. The injection device comprises the injection unit and further comprises an injection device data interface and an injection device housing. The supplementary device comprises the indication unit and further comprises a supplementary device data interface and an supplementary device housing. The injection device data interface is adapted to transmit an administration dose amount AD to the supplementary device data interface. Because the injection device only comprises a minimum number of components, the injection device may be rather slim, lightweight and cheap for this type of embodiment.

In all embodiments comprising an injection device and an supplementary device, the injection device is preferably adapted to be held in a hand while performing a drug administration and may especially be pen-shaped.

In all embodiments comprising an injection device and a supplementary device, the supplementary device may have the form of a Personal Digital Assistant (PDA) or a mobile phone. In some especially preferred embodiments, the supplementary device is a commercially available device such as a PDA or a cell phone.

In especially preferred embodiments comprising an injection device and a supplementary device, injection device, data interface and supplementary device data interface form a bidirectional communication interface. The bidirectional communication interface is especially preferably a wireless infrared interface and/or BLUETOOTH RF interface but may also be a wired interface, such as a Universal Serial Bus (USB) interface.

It is a second objective of the present invention to provide a method for setting a dose amount of a liquid drug for administration, the method preventing unintended deviation of an administration dose amount AD from a target dose amount TD.

The method for setting a dose amount according to the present invention comprises the steps of a) providing a target dose amount TD, b) operating manually operated dose setting means, wherein operating the manually operated dose setting means sets a an administration dose amount AD, and c) indicating by an electronic indication unit the relation between the administration dose amount AD and the target dose amount TD. Both step a) and step c) of the method for setting a dose according to the present invention are carried out by a correspondingly adapted device. The device may especially be an administration device according to the present invention

In preferred embodiments, step c) is performed in parallel with step b), such that the indication is provided while operating the dose setting means in real-time.

The relation between the administration dose amount AD and the target dose amount TD is preferably determined by electronic circuitry of the indication unit or by further electronics components such as a micro controller, an ASIC and/or further electronic circuitry known in the art.

In preferred embodiments, indicating the relation between the administration dose amount AD and the target dose amount TD comprises at least either of an optical indication, an acoustic indication and a tactile indication. In especially preferred embodiments, the method for setting a dose amount according to the present invention is carried out with an administration device according to the present invention with the indication being provided by the indication unit of the administration device.

In some cases, the administration dose amount AD deviates from the target dose amount TD intentionally. In the framework of insulin administration for the therapy of diabetes mellitus, this may be the case, e.g., when taking in carbohydrates immediately before, during or after sportive activities which generally result in a reduced insulin demand. In most cases, however, it is important to administer an administration dose amount AD which equals the target dose amount TD while administering an administration dose amount AD different from the target dose amount TD may happen by mistake. Therefore, the method for setting a dose amount preferably comprises the step of providing a warning if the administration dose amount AD is administered and the administration dose amount AD does not the equal the target dose amount TD. The warning preferably comprises at least one of an optical indication, an acoustical indication and a tactile indication and is provided by the indication unit of the indication device.

The method for setting a dose amount of a liquid drug according to the present invention is advantageously applied for the administration of insulin in the framework of diabetes mellitus therapy. It may, however be applied for the administration of other drugs, e.g. for the administration of pain relievers, cancer drugs, growth hormones, or the like.

In the following, exemplary administration devices according to the present invention and exemplary methods for setting a dose amount according to the present invention are described in greater detail with reference to the figures. All embodiments are described with respect to insulin administration, but may equally be applied for other the administration of other drugs, such as pain relievers, cancer drugs or growth hormones.
Figure 1 shows a first example of an administration device according to the present invention, the administration device being adapted to carry out a first exemplary method for setting a dose amount according to the present invention.
Figure 2 shows a structural diagram of the device shown in Figure 1.
Figure 3a and Figure 3b show an exemplary output of a display, the display belonging to the indication unit of an administration device according to Figure 1 and Figure 2.
Figure 4 shows a structural diagram of a second example of an administration device according to the present invention, the administration device being adapted to carry out a second exemplary method for setting a dose amount according to the present invention.

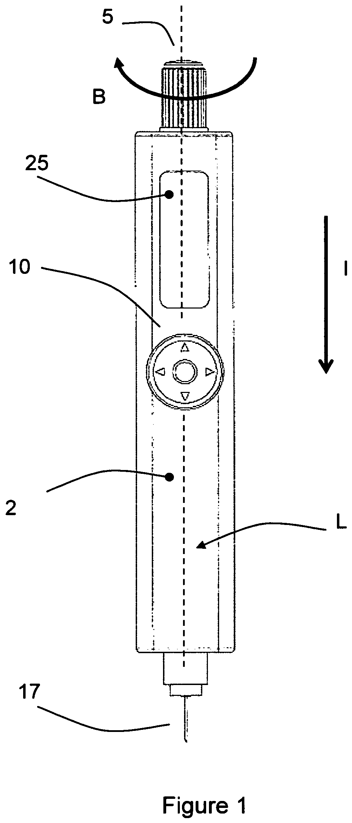

Figure 1 shows a first example of an administration device for the self administration of insulin in accordance with the invention and adapted to carry out a first exemplary method for the administration of a liquid in accordance with the present invention.

Most components of the administration device are enclosed by a pen-shaped housing 2. The Housing 2 comprises coupling means (not shown) for releasable coupling with an injection cannula 17, cannula 17 projecting from the housing 2 into the direction indicated by vector 1, vector 1 being parallel to longitudinal axis L of housing 2.

Cannula 17 is typically disposable and should be used for a single injection for sterility reasons. In other embodiments, cannula 17 may be used for several injections. A cover (not shown in Fig. 1) may be provided to cover the cannula coupling means or the cannula 17, respectively.

A Dose setting knob 5 serves as dose setting means for setting an insulin administration dose amount AD to be administered. Dose setting knob 5 is normally in a retracted position inside housing 2 and may be extended by pressing a release key (on the backside of housing 2, not visible). In its extended position, dose setting knob 5 may be rotated clockwise into rotational direction B for increasing an administration dose amount AD or may be rotated anti-clockwise (against rotational direction B) for decreasing an administration dose amount AD. The minimum dose amount increment is defined by rotational catching positions corresponding to, e.g., 0.5 IU (International Units) while a full rotation of dose setting knob 5 corresponds to, e.g. 20 IU. After setting the administration dose amount AD to be administered, the patient may grip the device with one hand and perform the injection by (i) piercing the skin of, e.g., an upper arm or thigh by moving the device into direction 1 towards the skin, and (ii) driving a dosing mechanism 6 to administer the insulin dose by slowly pressing dosing knob 5 down and back into its retracted position. The manually driven dosing mechanism may be designed according to the disclosure of WO 93/16740 or another suited design known in the art.

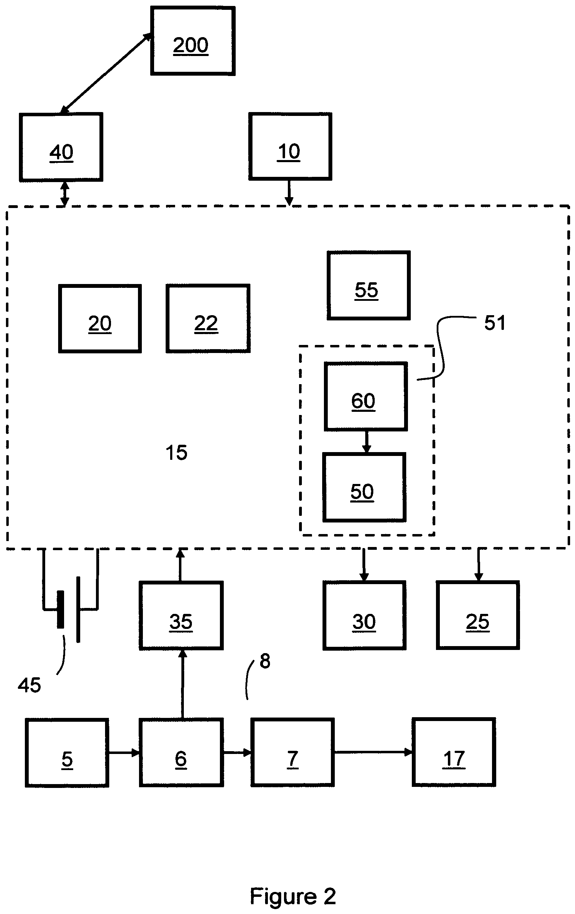

In the following, reference is made to Figure 1 as well as to Figure 2, Figure 2 showing a structural diagram of the device.

Dose setting knob 5 acts on dosing mechanism 6. When dose setting knob 5 is pressed down into its retracted position, dosing mechanism 6 forces the plunger of insulin cartridge 7 forward (into the direction indicated by vector 1), resulting in an insulin dose being administered via cannula 17. In combination, dose setting knob 5, dosing mechanism 6, and insulin cartridge 7 form the injection unit 8.

For scanning the rotational movement of dose setting knob 5, encoder 35 is provided which detects the rotation of dose setting knob 5 and transfers a corresponding electrical signal to controller 15 dependent on the direction of rotation. The encoder comprises a set of linear cam followers driven by a crank shaft, a set of cam discs or the like, the cam followers being operatively coupled to a corresponding set of electrical contacts. Similarly, optical encoders or magnetic encoders with magnets and hall sensors may be employed. The encoder may, e.g., be of the design disclosed in WO 93/16743.

The device includes an electronics module fulfilling multiple functions. The core element of the electronics module is controller 15 realized as Application Specific Integrated Circuit (ASIC). Most of the further elements of the electronics module are integral with controller 15.

Controller 15 is operatively coupled with an indication unit 25, 30. A LCD 25 is provided for showing numeric data and application specific data. When rotating dose setting knob 5, the dose amount is computed by controller 15 based on the signals generated by encoder 35 and is displayed on display 25.

Besides display 25, indication unit 25, 30 comprises a buzzer 30 especially for notification and indication purposes.

When actually administering insulin, the dose amount is stored in memory 20 together with a time stamp generated by clock circuit 22, the time stamp comprising a time of day portion and a date portion.

Data entry means 10 are especially suited for entering carbohydrate amount data but are also used for further data entry and control operations. In this exemplary embodiment, data entry means 10 are realized by a crossbar switch with an additional central ENTER key. This arrangement allows for an ergonomic one-handed operation.

The electronics module further comprises a power supply 45 in form of a replacable or non-replacable battery 45. The electronics module changes from standby to regular operation whenever dose setting knob 5 is extended or data entry means 10 are operated. The electronics module changes from regular operation to the standby state occur if no operation is being performed for a threshold time of, e.g. 2 minutes.

Carbohydrate amounts are preferably entered in carbohydrate exchange units which are used by many diabetics. However, other units such as grams of carbohydrates may be used, too. The device may be configured by the patient or the health care professional for carbohydrate amount entry in the desired units.

After entering a carbohydrate amount, the entry is confirmed and completed by pressing the ENTER key which results in the carbohydrate amount being stored in memory 20 together with a time stamp generated by clock circuit 22. If the patient does not modify the entered amount of carbohydrates nor presses the ENTER key within a certain time of, e.g. 5 sec, the entry is canceled.

Because the dose amount of an insulin dose required to compensate for food intake is directly correlated with the carbohydrate amount, the required calculated dose amount Dcalc can be automatically calculated based on the carbohydrate amount entered via data entry means 10. For this purpose, dose calculator 55 is provided by an algorithm on controller 15. The calculated dose amount Dcalc is calculated based on a first set of patient-specific and time-of-day dependent proportionality factors. The first set of proportionality factors is preferably uploaded to the electronics module from a data interface 40 but may also be entered via data entry means 10. The calculated dose amount Dcalc is stored in memory 20 along with the carbohydrate amount entered via data entry means 10.

After calculating the calculated dose amount Dcalc, it is displayed on display 25. The calculated dose amount Dcalc may either be directly accepted for administration or may be modified via data entry means 10. This may be necessary, e.g. in order to compensate for sportive activity or an undesirably high or low blood glucose value. Afterwards, an administration dose amount AD is manually set and administered using dose setting knob 5 as described above. While an administration dose amount AD different from the calculated dose amount Dcalc may be administered on purpose as an exception, it is normally important to ensure that the administration dose amount AD equals the calculated dose amount Dcalc. For this purpose, difference calculator 60 and zero detector 50 are provided, difference calculator 60 and zero detector 50 together forming dose comparator 51. The calculated dose amount Dcalc is fed into difference calculator 60 as the target dose amount TD, and difference calculator 60 calculates the dose amount deviation ΔD between the administration dose amount AD and the target dose amount TD. The dose amount deviation ΔD is fed into zero detector 50. Zero detector 50 indicates if dose amount deviation ΔMD is zero, indicating that the administration dose amount AD equals target dose amount TD.

Display 25 and optionally buzzer 30 are used to indicate if the administration dose amount AD equals the target dose amount TD and to indicate dose amount deviation ΔD.

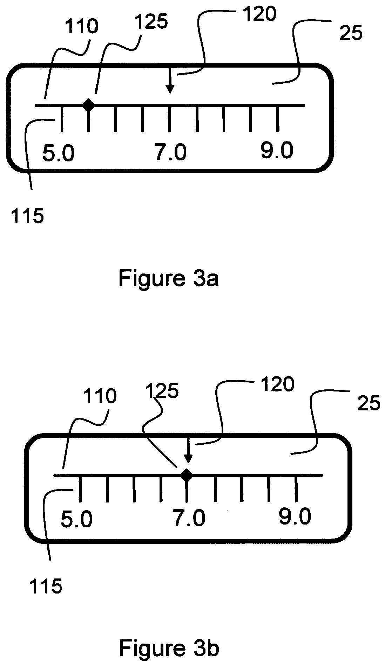

Figure 3a and Figure 3b show an exemplary output of display 25, assuming that a dose amountof Dcalc = 7.0IU of insulin have been calculated by dose calculator 55 to cover a carbohydrate amount entered via data entry means 10 and accordingly a target dose amount of TD = Dcalc = 7.0IU is fed into difference calculator 50 of dose comparator 51.

Display 25 shows a dose scale 110 with graduation 115. Scale 110 is calculated and arranged such that the target dose amount TD is located at the center of scale 110. The range of scale 110 is from TD - 2.0IU to TD + 2.0IE. The target dose amount TD is further indicated by an arrow-shaped target dose mark 120. An administration dose mark 125 indicates the administration dose amount AD. Administration dose mark 125 moves on scale 110 while rotating dose setting knob 5. Figure 3a shows the display for administration dose amount AD = 5.5IU, while Figure 3b shows the display for AD = TD = 7.0IU. In this latter case, target dose mark 120 and administration dose mark 125 are aligned. The alignment of administration dose mark 125 and target dose mark 120 would generally be sufficient to indicate the equivalence of administration dose amount AD and target dose amount TD. However, zero detector 50 is used to supply a further and more explicit indication. Preferably, target dose mark 120 blinks if administration dose amount AD does not equal target dose amount TD and is shown continuously if administration dose amount AD equals target dose amount TD. Alternatively or additionally, an acoustical indication may be provided via buzzer 30.

If administration dose amount AD is not in the range of scale 110, the numeric value of administration dose amount AD is shown on display 25 instead of scale 110. The numeric value of the target dose amount TD may be displayed additionally.

If administration dose amount AD is administered without administration dose amount AD being equal to target dose amount TD, an appropriate warning is shown on display 25 and/or an acoustic warning is provided via buzzer 30.

Comparing administration dose amount AD with target dose amount TD and controlling display 25 to indicate when administration dose amount AD equals target dose amount TD is performed in parallel with setting the administration dose amount AD by rotating dose setting knob 5 such that the difference between administration dose amount AD and target dose amount TD is displayed in real-time and the equivalence of administration dose amount AD and target dose amount TD is indicated in real-time when rotating dose setting knob 5.

In order to download the data stored in memory 20 to an external device 200, data interface 40 is provided. Data interface 40 is realized as wireless infrared interface and/or RF interface, especially a BLUETOOTH interface. For downloading purposes, a DOWNLOAD HISTORY function is provided which may be selected via data entry means 10. The external device may be any device such as a PC, a PDA or a cell phone.

Data interface 40 is not only used for history download, but may also be used to upload data from an external device to the administration device. It can especially be used to set configuration data such as time and date in clock circuit 22, the unit for entering carbohydrate amounts and proportionality factors for dose calculator 55. Furthermore, it may be used to upload a target dose amount TD from an external device 200 into memory 20. This kind of target dose amount TD may, e.g. be a dose required for covering the basal insulin need with an insulin dose of the same amount being administered every day and at the same time of day and may, e.g. by uploaded to the administration device by a healthcare professional.

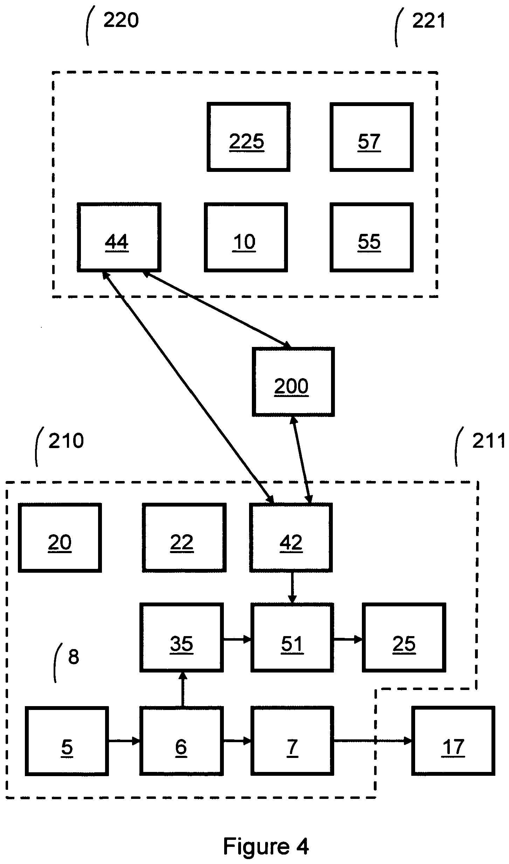

Figure 4 shows a structural diagram of a second example of a device for the self administration of insulin in accordance with the invention and adapted to carry out a second exemplary method for the administration of a liquid in accordance with the present invention.

This exemplary device comprises an injection device 210 and an supplementary device 220. Injection unit 8, an injection device data interface 42, dose comparator 51 and indication unit 25' belong to the injection system and are enclosed by an injection device housing 211. Injection device housing 211 has a substantially pen-shaped form, similar to the exemplary device shown in Figure 1. Data entry means 10, dose calculator 55 and a supplementary device data interface 44 belong to an supplementary device 220 and are enclosed by an supplementary device housing 221.

Supplementary device housing 221 is slim and substantially box-like, similar to a PDA.

Even though not explicitly shown in Figure 4, supplementary device 220 comprises further components such as a power supply, memory, clock circuit, data out put means and the like.

After entering a carbohydrate amount via data entry means 10, the corresponding calculated dose amount Dcalc is calculated by dose calculator 55 and transmitted from supplementary device data interface 44 to injection device data interface 42. Dose setting is subsequently performed via dose setting knob 5 as described above for the first exemplary device.

Though display 25 may be identical to the fist exemplary embodiment, it is also possible to use a considerably simpler display such as a pure numerical display with additional symbols to indicate if administration dose amount AD equals target dose amount TD. The possibility of providing a display 25 with rather low performance in the second exemplary embodiment is due to he fact that display 25 is not being used to show further data such as therapy related data in contrast to the first exemplary embodiment. Alternatively, a mechanical display operatively coupled to dose setting knob 5 may be used to display administration dose amount AD and a different optical indication unit comprising, e.g., LEDs and/or buzzer 30 may be provided to indicate when administration dose amount AD equals target dose amount TD.

Providing data entry means 10 as part of an supplementary device 220 allows the injection device housing 211 to be especially slim and lightweight. Furthermore, it allows to design data entry means 10 especially comfortable and user-friendly. Data entry means 10 is designed as touch-screen in this exemplary embodiment, but may also have the form of a crossbar switch, a data entry wheel, a keyboard, or the like, or any combination of such elements.

Supplementary device 220 further encloses a carbohydrate amount database 57. Carbohydrate amount database stores 57 the relative and/or absolute carbohydrate amounts of a number of meals and is operatively coupled to dose calculator 55.

Typical and/or frequently taken meals may be comfortably selected from carbohydrate amount database 57 without requiring the direct entry of carbohydrate amounts.

Supplementary device 220 further comprises a glucose meter 225 for the self-determination of blood glucose values. Glucoses meter 225 employs disposable single-use test strips as well known in the art. Glucose meter 225 is operatively coupled to dose calculator 55. In order to calculate a calculated dose amount Dcalc for an insulin dose dose required to compensate for undesirably high blood glucose values, a second set of patient-dependent and time-of-day dependent proportionality factors specifying the dose amount of an insulin dose required to correct a given raise in blood glucose is provided. Based on this second set of proportionality factors, dose calculator 55 automatically calculates the appropriate insulin dose amount Dcalc. This dose is afterwards transmitted from supplementary device data interface 44 to injection device data interface 32. As for insulin doses for covering carbohydrate intake, calculated dose amount Dcalc is fed as target dose amount TD into dose comparator 51 and administration dose amount AD is set via dose setting knob 5. Because blood glucose measurements and the administration of insulin for the compensation of undesirably high blood glucose values are often performed directly prior to a meal, a combined calculated dose amount Dcalc may be calculated, taking into account both the insulin required to compensate for an undesired high blood glucose value and the carbohydrate intake. If the blood glucose value is undesirably low, the combined calculated dose Dcalc may also be smaller than the dose amounts that would be calculated without considering the blood glucose value.

Alternatively or additional to glucose meter 225, supplementary device 220 may receive blood glucose data from an external blood glucose meter via supplementary device data interface 44 or and additional data interface. It is further possible to determine a blood glucose value using a blood glucose meter and manually enter the determined blood glucose value using data entry means 10.

In this exemplary embodiment, injection device data interface 42 and supplementary device data interface 44 are designed for bidirectional communication according to a common standard, especially via infrared. Data stored in memory 20 may be directly downloaded to an external device 200 via injection device data interface 42. Alternatively, data stored in memory 20 may be first transmitted to supplementary device 220 via injection device data interface 42 and supplementary device data interface 44 and afterwards be transmitted to from supplementary device 220 to external device 200 via supplementary device data interface 200. Supplementary device data interface 44 comprise multiple interface means. Supplementary device data interface 44 is especially designed for communication with injection device data interface 42 via infrared while it enables communication with external device 200 via more powerful means such as BLUETOOTH and/or a wired Universal Serial Bus (USB).

Even though the first exemplary embodiment and the second exemplary embodiment may be completely separated and use different types of devices, it is also possible to combine some of the elements of the first exemplary embodiment and the second exemplary embodiment. More particularly, an administration device according to the first exemplary embodiment may be used as stand-alone device in situations where it is undesirable to carry an supplementary device 220, whereas an supplementary device 220 offers further advantages such as a more comfortable data entry means 10 and carbohydrate amount database 57 when both devices are used in combination.

## Claims

1. Administration device for the administration of a liquid drug in adjustable doses, comprising:
a) an injection unit (8), the injection unit (8) comprising
• a manually operated dose setting means (5), wherein operating the manually operated dose setting means (5) sets an administration dose amount AD, and
• a manually driven dosing mechanism (6),
b) an electronic indication unit (25, 30) and electronics circuitry, **characterized in** the electronics circuitry determining a relation between the administration dose amount AD and a target dose amount TD, the target dose amount TD being a dose amount that is intended for administration, the indication unit (25, 30) indicating a relation between the administration dose amount AD and the target dose amount TD,
the target dose amount TD being transmitted to the administration device from an external device via a data interface of the administration device or being calculated by a dose calculator of the administration device based on therapy-related data or being stored in the administration device as several alternative values for manual or automatic selection.

2. Administration device according to either of the previous claims, **characterized in that** the indication unit comprises a display (25), the display indicating at least one of the administration dose amount AD, the target dose amount TD and a dose amount deviation ΔD, with the dose amount deviation Δ D indicating the deviation between the administration dose amount AD and the target dose amount TD.

3. Administration device according to claim 2, **characterized that** the display (25) displays a graphical representation of the dose amount deviation ΔD.

4. Administration device according to either of the preceding claims, wherein the indication of the relation between the administration dose amount AD and a target dose amount TD is provided in parallel with setting the administration dose amount AD.

5. Administration device according to either of the previous claims, **characterized in that** the indication unit (25, 30) comprises at least one of an acoustical indicator (30) and a tactile indicator.

6. Administration device according to either of the previous claims, **characterized in that** the administration device comprises an injection device (210) and an supplementary device (220),
• the injection device (210) comprising the injection unit (8), the indication unit (25'), an injection device data interface (42) and an injection device housing (211),
• the supplementary device (220) comprising a supplementary device data interface (44) and a supplementary device housing (221),
• wherein the supplementary device data interface (44) is adapted to transmit the target dose amount TD to the injection device data interface (42).

7. Administration device according to either of either of the previous claims, **characterized in that** the administration device comprises an injection device (210) and an supplementary device (220),
• the injection device (210) comprising the injection unit (8), an injection device data interface (42) and an injection device housing (211),
• the supplementary device (220) comprising the indication unit (25), a supplementary device data interface (44) and an supplementary device housing (221),
• wherein the injection device data interface (44) is adapted to transmit an administration dose amount AD to the supplementary device data interface (42).

8. Administration device according to either of the previous claims, **characterized in that** the administration device further comprises a data entry means (10).

9. Administration device according to either of the previous claims, **characterized in that** the therapy related data comprise at least one of a carbohydrate amount or a blood glucose value.

10. Administration device according to claim 9, **characterized in that** the administration device further comprises a carbohydrate amount database (57), the carbohydrate amount database (57) storing the relative and/or absolute carbohydrate amounts of a number of meals, the carbohydrate amount database (57) being operatively coupled to the dose calculator (55), wherein meals may be selected from the carbohydrate amount database (57) via data entry means (10).

11. Administration device according to either of the previous claims, **characterized in that** the administration device further comprises a blood glucose meter (225).

12. Administration device according to either of the previous claims, **characterized in that** the administration device is designed to be handheld while performing drug administration.

13. Method for setting a dose of a liquid drug for administration, comprising the steps of:
a) providing a target dose amount TD, the target dose amount TD being a dose amount that is intended for administration, the target dose amount TD being transmitted to the administration device form an external device via a data interface of the administration device or being calculated by a dose calculator of the administration device based on therapy-related data or being stored in the administration device as several alternative values for manual or automatic selection;
b) operating a manually operated dose setting means (5), wherein operating the manually operated dose setting means sets an administration dose amount AD, and
c) determining by electronics circuitry a relation between the administration dose amount AD and the target dose amount TD, and indicating by an electronic indication unit (25, 30) the relation between the administration dose amount AD and the target dose amount TD.

14. Method according to claim 13, the method including indicating by a display (25) of the electronic indication unit at least one of the administration dose amount AD, the target dose amount TD and a dose amount deviation Δ D, with the dose amount deviation ΔD indicating the deviation between the administration dose amount AD and the target dose amount TD.

## Patentansprüche

1. Verabreichungsvorrichtung zur Verabreichung eines flüssigen Arzneimittels in einstellbaren Dosen, umfassend:
a) eine Injektionseinheit (8), wobei die Injektionseinheit (8) Folgendes umfasst
• eine manuell bediente Dosiseinstellungseinrichtung (5), wobei das Bedienen der manuell bedienten Dosiseinstellungseinrichtung (5) eine Verabreichungsdosismenge AD einstellt, und
• einen manuell angetriebenen Dosierungsmechanismus (6),
b) eine elektronische Anzeigeeinheit (25, 30) und eine Elektronikschaltung, **dadurch gekennzeichnet, dass** die Elektronikschaltung eine Beziehung zwischen der Verabreichungsdosismenge AD und einer Zieldosismenge TD bestimmt, wobei die Zieldosismenge TD eine Dosismenge ist, die zur Verabreichung vorgesehen ist, wobei die Anzeigeeinheit (25, 30) eine Beziehung zwischen der Verabreichungsdosismenge AD und der Zieldosismenge TD anzeigt,
wobei die Zieldosismenge TD von einer externen Vorrichtung über eine Datenschnittstelle der Verabreichungsvorrichtung an die Verabreichungsvorrichtung übermittelt oder basierend auf therapiebezogenen Daten von einem Dosisberechner der Verabreichungsvorrichtung berechnet oder als mehrere alternative Werte für manuelle oder automatische Auswahl in der Verabreichungsvorrichtung gespeichert wird.

2. Verabreichungsvorrichtung nach einem der vorstehenden Ansprüche, **dadurch gekennzeichnet, dass** die Anzeigeeinheit ein Display (25) umfasst, wobei das Display mindestens eines von der Verabreichungsdosismenge AD, der Zieldosismenge TD und einer Dosismengenabweichung ΔD anzeigt, wobei die Dosismengenabweichung ΔD die Abweichung zwischen der Verabreichungsdosismenge AD und der Zieldosismenge TD anzeigt.

3. Verabreichungsvorrichtung nach Anspruch 2, **dadurch gekennzeichnet, dass** das Display (25) eine graphische Darstellung der Dosismengenabweichung ΔD zeigt.

4. Verabreichungsvorrichtung nach einem der vorstehenden Ansprüche, wobei die Anzeige der Beziehung zwischen der Verabreichungsdosismenge AD und einer Zieldosismenge TD parallel zur Einstellung der Verabreichungsdosismenge AD bereitgestellt wird.

5. Verabreichungsvorrichtung nach einem der vorstehenden Ansprüche, **dadurch gekennzeichnet, dass** die Anzeigeeinheit (25, 30) mindestens einen von einem akustischen Indikator (30) und einem taktilen Indikator umfasst.

6. Verabreichungsvorrichtung nach einem der vorstehenden Ansprüche, **dadurch gekennzeichnet, dass** die Verabreichungsvorrichtung eine Injektionsvorrichtung (210) und eine Zusatzvorrichtung (220) umfasst,
• wobei die Injektionsvorrichtung (210) die Injektionseinheit (8), die Anzeigeeinheit (25'), eine Injektionsvorrichtungsdatenschnittstelle (42) und ein Injektionsvorrichtungsgehäuse (211) umfasst,
• wobei die Zusatzvorrichtung (220) eine Zusatzvorrichtungsdatenschnittstelle (44) und ein Zusatzvorrichtungsgehäuse (221) umfasst,
• wobei die Zusatzvorrichtungsdatenschnittstelle (44) zum Übermitteln der Zieldosismenge TD an die Injektionsvorrichtungsdatenschnittstelle (42) ausgelegt ist.

7. Verabreichungsvorrichtung nach einem der vorstehenden Ansprüche, **dadurch gekennzeichnet, dass** die Verabreichungsvorrichtung eine Injektionsvorrichtung (210) und eine Zusatzvorrichtung (220) umfasst,
• wobei die Injektionsvorrichtung (210) die Injektionseinheit (8), eine Inj ektionsvorrichtungsdatenschnittstelle (42) und ein Injektionsvorrichtungsgehäuse (211) umfasst,
• wobei die Zusatzvorrichtung (220) die Anzeigeeinheit (25), eine Zusatzvorrichtungsdatenschnittstelle (44) und ein Zusatzvorrichtungsgehäuse (221) umfasst,
• wobei die Injektionsvorrichtungsdatenschnittstelle (44) zum Übermitteln einer Verabreichungsdosismenge AD an die Zusatzvorrichtungsdatenschnittstelle (42) ausgelegt ist.

8. Verabreichungsvorrichtung nach einem der vorstehenden Ansprüche, **dadurch gekennzeichnet, dass** die Verabreichungsvorrichtung ferner eine Dateneingabeeinrichtung (10) umfasst.

9. Verabreichungsvorrichtung nach einem der vorstehenden Ansprüche, **dadurch gekennzeichnet, dass** die therapiebezogenen Daten mindestens eines von einer Kohlenhydratmenge oder einem Blutzuckerwert umfassen.

10. Verabreichungsvorrichtung nach Anspruch 9, **dadurch gekennzeichnet, dass** die Verabreichungsvorrichtung ferner eine Kohlenhydratmengendatenbank (57) umfasst, wobei die Kohlenhydratmengendatenbank (57) die relativen und/oder absoluten Kohlenhydratmengen mehrerer Mahlzeiten speichert, wobei die Kohlenhydratmengendatenbank (57) funktional an den Dosisberechner (55) gekoppelt ist, wobei Mahlzeiten über eine Dateneingabeeinrichtung (10) aus der Kohlenhydratmengendatenbank (57) ausgewählt werden können.

11. Verabreichungsvorrichtung nach einem der vorstehenden Ansprüche, **dadurch gekennzeichnet, dass** die Verabreichungsvorrichtung ferner ein Blutzuckermessgerät (225) umfasst.

12. Verabreichungsvorrichtung nach einem der vorstehenden Ansprüche, **dadurch gekennzeichnet, dass** die Verabreichungsvorrichtung so konstruiert ist, dass sie während der Arzneimittelverabreichung in der Hand gehalten werden kann.

13. Verfahren zum Einstellen einer Dosis eines flüssigen Arzneimittels zur Verabreichung, umfassend die folgenden Schritte:
a) Bereitstellen einer Zieldosismenge TD, wobei die Zieldosismenge TD eine Dosismenge ist, die zur Verabreichung vorgesehen ist, wobei die Zieldosismenge TD von einer externen Vorrichtung über eine Datenschnittstelle der Verabreichungsvorrichtung an die Verabreichungsvorrichtung übermittelt oder basierend auf therapiebezogenen Daten von einem Dosisberechner der Verabreichungsvorrichtung berechnet oder als mehrere alternative Werte für manuelle oder automatische Auswahl in der Verabreichungsvorrichtung gespeichert wird;
b) Bedienen einer manuell bedienten Dosiseinstellungseinrichtung (5), wobei das Bedienen der manuell bedienten Dosiseinstellungseinrichtung eine Verabreichungsdosismenge AD einstellt, und
c) Bestimmen einer Beziehung zwischen der Verabreichungsdosismenge AD und der Zieldosismenge TD mittels der Elektronikschaltung und Anzeigen der Beziehung zwischen der Verabreichungsdosismenge AD und der Zieldosismenge TD mittels einer elektronischen Anzeigeeinheit (25, 30).

14. Verfahren nach Anspruch 13, wobei das Verfahren das Anzeigen mindestens eines von der Verabreichungsdosismenge AD, der Zieldosismenge TD und einer Dosismengenabweichung ΔD mittels eines Displays (25) der elektronischen Anzeigeeinheit einschließt, wobei die Dosismengenabweichung ΔD die Abweichung zwischen der Verabreichungsdosismenge AD und der Zieldosismenge TD anzeigt.

## Revendications

1. Dispositif d'administration pour l'administration d'un médicament liquide en doses ajustables, comprenant :
a) une unité d'injection (8), l'unité d'injection (8) comprenant
• un moyen de réglage de dose actionné manuellement (5), dans lequel l'actionnement du moyen de réglage de dose actionné manuellement (5) règle une quantité de dose d'administration AD, et
• un mécanisme de dosage entraîné manuellement (6),
b) une unité d'indication électronique (25, 30) et un circuit électronique, **caractérisé en ce que** le circuit électronique déterminant une relation entre la quantité de dose d'administration AD et une quantité de dose cible TD, la quantité de dose cible TD étant une quantité de dose qui est destinée à l'administration, l'unité d'indication (25, 30) indiquant une relation entre la quantité de dose d'administration AD et la quantité de dose cible TD,
la quantité de dose cible TD étant transmise au dispositif d'administration à partir d'un dispositif externe par le biais d'une interface de données du dispositif d'administration ou étant calculée par un calculateur de dose du dispositif d'administration sur la base de données liées à une thérapie ou étant stockée dans le dispositif d'administration en tant que plusieurs valeurs alternatives pour une sélection manuelle ou automatique.

2. Dispositif d'administration selon l'une quelconque des revendications précédentes, **caractérisé en ce que** l'unité d'indication comprend un affichage (25), l'affichage indiquant au moins un parmi la quantité de dose d'administration AD, la quantité de dose cible TD et un écart de quantité de dose ΔD, l'écart de quantité de dose ΔD indiquant l'écart entre la quantité de dose d'administration AD et la quantité de dose cible TD.

3. Dispositif d'administration selon la revendication 2, **caractérisé en ce que** l'affichage (25) affiche une représentation graphique de l'écart de quantité de dose ΔD.

4. Dispositif d' administration selon l'une quelconque des revendications précédentes, dans lequel l'indication de la relation entre la quantité de dose d'administration AD et une quantité de dose cible TD est fournie en parallèle avec le réglage de la quantité de dose d'administration AD.

5. Dispositif d'administration selon l'une quelconque des revendications précédentes, **caractérisé en ce que** l'unité d'indication (25, 30) comprend au moins un parmi un indicateur acoustique (30) et un indicateur tactile.

6. Dispositif d'administration selon l'une quelconque des revendications précédentes, **caractérisé en ce que** le dispositif d'administration comprend un dispositif d'injection (210) et un dispositif supplémentaire (220),
• le dispositif d'injection (210) comprenant l'unité d'injection (8), l'unité d'indication (25'), une interface de données de dispositif d'injection (42) et un boîtier de dispositif d'injection (211),
• le dispositif supplémentaire (220) comprenant une interface de données de dispositif supplémentaire (44) et un boîtier de dispositif supplémentaire (221),
• dans lequel l'interface de données de dispositif supplémentaire (44) est adaptée pour transmettre la quantité de dose cible TD à l'interface de données de dispositif d'injection (42).

7. Dispositif d'administration selon l'une quelconque des revendications précédentes, **caractérisé en ce que** le dispositif d'administration comprend un dispositif d'injection (210) et un dispositif supplémentaire (220),
• le dispositif d'injection (210) comprenant l'unité d'injection (8), une interface de données de dispositif d'injection (42) et un boîtier de dispositif d'injection (211),
• le dispositif supplémentaire (220) comprenant l'unité d'indication (25), une interface de données de dispositif supplémentaire (44) et un boîtier de dispositif supplémentaire (221),
• dans lequel l'interface de données de dispositif d'injection (44) est adaptée pour transmettre une quantité de dose d'administration AD à l'interface de données de dispositif supplémentaire (42).

8. Dispositif d'administration selon l'une quelconque des revendications précédentes, **caractérisé en ce que** le dispositif d'administration comprend en outre un moyen d'entrée de données (10).

9. Dispositif d'administration selon l'une quelconque des revendications précédentes, **caractérisé en ce que** les données liées à une thérapie comprennent au moins une parmi une quantité de glucides ou une glycémie.

10. Dispositif d'administration selon la revendication 9, **caractérisé en ce que** le dispositif d'administration comprend en outre une base de données de quantités de glucides (57), la base de données de quantités de glucides (57) stockant les quantités de glucides relatives et/ou absolues d'un certain nombre de repas, la base de données de quantités de glucides (57) étant couplée de manière fonctionnelle au calculateur de dose (55), dans lequel les repas peuvent être sélectionnés à partir de la base de données de quantités de glucides (57) par le biais du moyen d'entrée de données (10).

11. Dispositif d'administration selon l'une quelconque des revendications précédentes, **caractérisé en ce que** le dispositif d'administration comprend en outre un glucomètre (225).

12. Dispositif d'administration selon l'une quelconque des revendications précédentes, **caractérisé en ce que** le dispositif d'administration est conçu pour être tenu dans la main pendant la réalisation de l'administration de médicament.

13. Procédé de réglage d'une dose d'un médicament liquide pour une administration, comprenant les étapes de :
a) fourniture d'une quantité de dose cible TD, la quantité de dose cible TD étant une quantité de dose qui est destinée à l'administration, la quantité de dose cible TD étant transmise au dispositif d'administration à partir d'un dispositif externe par le biais d'une interface de données du dispositif d'administration ou étant calculée par un calculateur de dose du dispositif d'administration sur la base de données liées à une thérapie ou étant stockée dans le dispositif d'administration en tant que plusieurs valeurs alternatives pour une sélection manuelle ou automatique ;
b) actionnement d'un moyen de réglage de dose actionné manuellement (5), dans lequel l'actionnement du moyen de réglage de dose actionné manuellement règle une quantité de dose d'administration AD, et
c) détermination par le circuit électronique d'une relation entre la quantité de dose d'administration AD et la quantité de dose cible TD, et indication par une unité d'indication électronique (25, 30) de la relation entre la quantité de dose d'administration AD et la quantité de dose cible TD.

14. Procédé selon la revendication 13, le procédé incluant l'indication par un affichage (25) de l'unité d'indication électronique d'au moins un parmi la quantité de dose d'administration AD, la quantité de dose cible TD et un écart de quantité de dose ΔD, l'écart de quantité de dose ΔD indiquant l'écart entre la quantité de dose d'administration AD et la quantité de dose cible TD.
